(19) Europäisches Patentamt
European Patent Office
Office européen des brevets

(11) Numéro de publication: **0 321 952 B1**

(12) **FASCICULE DE BREVET EUROPEEN**

(45) Date de publication de fascicule du brevet: **19.08.92**

(51) Int. Cl.$^5$: **A61K 7/06**

(21) Numéro de dépôt: **88121401.9**

(22) Date de dépôt: **21.12.88**

Le dossier contient des informations techniques présentées postérieurement au dépôt de la demande et ne figurant pas dans le présent fascicule.

# (54) Association de dérivés de pyrimidine et d'agents anti-inflammatoires non stéroidiens pour induire et stimuler la croissance des cheveux et diminuer leur chute.

(30) Priorité: **22.12.87 LU 87090**

(43) Date de publication de la demande:
**28.06.89 Bulletin 89/26**

(45) Mention de la délivrance du brevet:
**19.08.92 Bulletin 92/34**

(84) Etats contractants désignés:
**AT BE CH DE FR GB GR IT LI NL SE**

(56) Documents cités:
**EP-A- 0 220 118**
**WO-A-88/07361**
**US-A- 4 141 976**

**S.T.N. FILE SUPPLIER, Karlsruhe (DE)**

**PATENT ABSTRACTS OF JAPAN, vol. 11, no. 115 (C-415)[2562], 10 avril 1987, page 145 C 415**

(73) Titulaire: **L'OREAL**
**14, Rue Royale**
**F-75008 Paris(FR)**

(72) Inventeur: **Grollier, Jean-Francois**
**16 Bis Boulevard Morland**
**F-75004 Paris(FR)**
Inventeur: **Rosenbaum, Georges**
**2, Rue J.H. Mansart**
**F-92600 Asnières(FR)**

(74) Mandataire: **Casalonga, Axel et al**
**BUREAU D.A. CASALONGA - JOSSE Morassistrasse 8**
**W-8000 München 5(DE)**

**EP 0 321 952 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

Rank Xerox (UK) Business Services

## Description

L'invention est relative à l'association de dérivés de pyrimidine et d'agents anti-inflammatoires non stéroïdiens en vue d'induire et de stimuler la croissance des cheveux et diminuer leur chute.

L'activité des follicules pileux est cyclique. A la phase anagène active, qui dure plusieurs années et au cours de laquelle le cheveu s'allonge, succède une phase de repos (télogène) de quelques mois. A la fin de cette période de repos, le cheveu tombe et un autre cycle recommence.

La chevelure se renouvelle donc en permanence; sur les 100.000 à 150.000 cheveux que comporte une chevelure, à chaque instant 10% environ sont au repos et seront donc remplacés en quelques mois.

Dans presque tous les cas, la chute des cheveux survient chez des sujets prédisposés génétiquement; elle atteint plus particulièrement les hommes.

Cette alopécie est une perturbation du renouvellement capillaire qui entraîne, dans un premier temps, une accélération de la fréquence des cycles aux dépens de la qualité des cheveux puis de leur quantité. Il y a un appauvrissement progressif de la chevelure par régression d'une partie des cheveux dits "terminaux" au stade de "duvets". Des zones sont touchées préférentiellement : golfes temporaux-frontaux, ... chez les hommes; alopécie diffuse du vertex chez les femmes.

Dans certaines dermatoses du cuir chevelu à caractéristique inflammatoire telles que par exemple le psoriasis ou les dermatites séborrhéiques, la chute des cheveux peut être fortement accentuée ou entraîner des cycles des follicules fortement perturbés.

On a déjà proposé dans le passé d'utiliser des composés tels que l'amino-6 dihydro-1,2 hydroxy-1 imino-2 pipéridino-4 pyrimidine ou encore "Minoxidil" dans des compositions permettant de réduire ou de supprimer l'effet de l'alopécie et d'induire ou de stimuler la croissance des cheveux et diminuer leur chute.

Des compositions topiques pour le traitement de la chute des cheveux contenant le Minoxidil en présence d'agents anti-inflammatoires tels que la glycyrrhyzine ou l'ibuprofene ont été décrites dans les demandes de brevet JP 260010/86 et WO 88107361.

La demanderesse a découvert maintenant, qu'en associant certains agents anti-inflammatoires non stéroïdiens avec certains dérivés de pyrimidine et plus particulièrement le "Minoxidil", on constate de façon surprenante une action et une stimulation améliorées de la croissance des cheveux et une action plus importante sur le freinage de la chute des cheveux. Ceci est particulièrement surprenant lorsqu'on sait que les agents anti-inflammatoires non stéroïdiens n'ont à priori aucune action sur le cycle pilaire.

La demanderesse a constaté, en particulier, que l'association avait une activité supérieure par rapport aux dérivés de pyrimidine utilisés seuls et que cette action était également plus rapide grâce à l'utilisation de l'association.

Cette association permet, notamment, d'utiliser les dérivés de pyrimidine à une concentration plus faible.

La demanderesse a découvert que certains anti-inflammatoires non stéroïdiens étaient particulièrement appropriés pour être utilisés en association avec le minoxidil ou ses dérivés en application topique, notamment en vue d'un traitement à long terme. Les composés retenus sont particulièrement peu toxiques, présentent en association avec le minoxidil une conservation améliorée, notamment en milieu anhydre et ont, par ailleurs, un effet de cosolubilisation, notamment en milieu anhydre, sur les dérivés de pyrimidine, tels que le minoxidil.

Pour déterminer l'efficacité ou la rapidité d'action d'une composition de traitement de l'alopécie, on utilise généralement le trichogramme et en particulier le photo-trichogramme qui permet de déterminer entre autre le pourcentage de cheveux en phase anagène par rapport aux cheveux en phase télogène.

Un objet de l'invention est donc constitué par l'association de dérivés de pyrimidine et d'agents anti-inflammatoires non stéroïdiens en vue d'induire ou de stimuler la croissance des cheveux et diminuer leur chute.

Un autre objet de l'invention est constitué par des compositions cosmétiques et/ou pharmaceutiques contenant une telle association.

L'invention vise également des dispositifs à plusieurs compartiments encore appelés "kits" ou trousses de traitement, comportant les différents composants de l'association.

D'autres objets de l'invention apparaîtront à la lecture de la description et des exemples qui suivent.

L'association conforme à l'invention est essentiellement caractérisée par le fait qu'elle comprend :

a/ un composant (A) contenant, dans un milieu physiologiquement acceptable, au moins un agent anti-inflammatoire non stéroïdien, choisi parmi les oxicams, l'acide niflumique, le diclofenac, le diflunisal, l'acide flufénamique, le bufexamac, le fenbufène, le fénoprofène.

b/ un composant (B) contenant dans un milieu physiologiquement acceptable au moins un dérivé de pyrimidine, répondant à la formule :

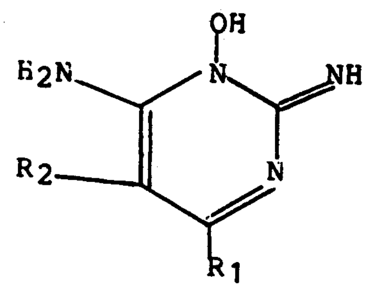

(I)

dans laquelle $R_1$ désigne un groupement

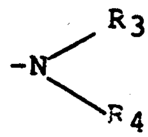

,

$R_3$, $R_4$, indépendamment l'un de l'autre, désignant hydrogène, un groupement alkyle, alcényle, alkylaryle, cycloalkyle, $R_3$ et $R_4$ pouvant également former un hétérocycle avec l'atome d'azote auquel ils sont liés, choisi parmi les groupements aziridinyle, azétidinyle, pyrrolidinyle, pipéridinyle, hexahydroazépinyle, heptaméthylèneimine, octaméthylèneimine, morpholine et alkyl(inférieur)-4 pipérazidinyle, les groupements hétérocycliques pouvant être substitués sur les atomes de carbone par un à trois groupements alkyle inférieur, hydroxy ou alcoxy; le groupement $R_2$ est choisi parmi un atome d'hydrogène, un groupement alkyle, alcényle, alkylalcoxy, cycloalkyle, aryle, alkylaryle, arylalkyle, alkylarylalkyle, alcoxyarylalkyle ou haloarylalkyle, ainsi que les sels d'addition d'acides physiologiquement acceptables, les composants (A) et (B) faisant partie d'une même composition ou étant destinés à être utilisés séparément, soit simultanément, soit de façon successive ou décalée dans le temps en vue d'induire et de stimuler la croissance des cheveux et diminuer leur chute.

Parmi les oxicams, on peut citer en particulier le Piroxicam.

Dans les composés de formule (I), les groupements alkyle ou alcoxy désignent de préférence un groupement ayant 1 à 4 atomes de carbone; le groupement alcényle désigne de préférence un groupement ayant 2 à 5 atomes de carbone; le groupement aryle désigne de préférence phényle et le groupement cycloalkyle désigne de préférence un groupement ayant 4 à 6 atomes de carbone.

Les composés préférés de formule (I) sont plus particulièrement choisis parmi les composés dans lesquels $R_2$ désigne hydrogène et $R_1$ représente un groupement :

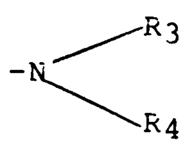

,

dans lequel $R_3$ et $R_4$ forment un cycle pipéridinyle, ainsi que leurs sels, tels que, par exemple, le sulfate.

Parmi ces composés, le composé particulièrement préféré est constitué par l'amino-6 dihydro-1,2 hydroxy-1 imino-2 pipéridino-4 pyrimidine, encore appelé "Minoxidil".

L'agent anti-inflammatoire particulièrement préféré est l'acide niflumique.

Les anti-inflammatoires non stéroïdiens définis ci-dessus sont utilisés dans le composant (A) dans des proportions comprises entre 0,01 et 5% en poids, de préférence entre 0,05 et 3% en poids et plus particulièrement entre 0,05 et 2% en poids; le dérivé de pyrimidine de formule (I) est utilisé dans le composant (B) dans des proportions comprises entre 0,05 et 10% en poids, de préférence entre 0,05 et 5% en poids et en particulier entre 0,5 et 4% en poids.

L'agent anti-inflammatoire non stéroïdien, défini ci-dessus, lorsque les composants (A) et (B) sont dans une même composition, est utilisé dans des proportions comprises entre 0,01 et 3% en poids par rapport au poids total de la composition, de préférence entre 0,02 et 2% en particulier entre 0,02 et 1%.

Le dérivé de pyrimidine de formule (I) est utilisé dans ce cas dans les compositions dans une proportion comprise entre 0,05 et 6% en poids, par rapport au poids total de la composition, de préférence

entre 0,1 et 5% en poids et en particulier entre 0,5 et 3% en poids.

Le milieu physiologiquement acceptable utilisé pour les composants (A) et (B) est un milieu utilisable en formulation cosmétique et peut être constitué par de l'eau, un mélange d'eau et d'un ou de plusieurs solvants ou par un ou plusieurs solvants. Les solvants sont des solvants organiques, acceptables en pharmacie ou en cosmétique.

Le milieu particulièrement préféré est un milieu anhydre c'est-à-dire contenant moins de 1% d'eau.

Les solvants plus particulièrement préférés sont choisis parmi les alcools inférieurs en $C_1$-$C_4$, tels que l'alcool éthylique, l'alcool isopropylique, l'alcool tertiobutylique; les alkylèneglycols comme le propylèneglycol; des alkyléthers de mono- et de dialkylèneglycol, dans lesquels les groupes alkyle et alkylène ont de préférence 1 à 4 atomes de carbone tels que plus particulièrement le monoéthyléther d'éthylèneglycol, le monométhyléther de propylèneglycol et le monoéthyléther de diéthylèneglycol.

Le milieu anhydre est constitué de préférence par un alcool inférieur en $C_1$-$C_4$ ou un mélange d'alcool en $C_1$-$C_4$ et d'un alkylèneglycol.

Les milieux physiologiquement acceptables peuvent être épaissis ou non et on peut utiliser pour épaissir des agents épaississants et/ou gélifiants bien connus dans l'état de la technique, tels que plus particulièrement les hétérobiopolysaccharides tels que la gomme de xanthane ou les scléroglucanes, les dérivés de cellulose, les polymères acryliques réticulés ou non.

Les solvants, lorsqu'ils sont utilisés dans un milieu aqueux, sont présents de préférence dans des proportions comprises entre 1 et 80% en poids par rapport au poids total de la composition ou de chacun des composants.

Les épaississants sont utilisés de préférence dans des proportions comprises entre 0,1 et 5% en poids, et en particulier entre 0,4 et 3% en poids, par rapport au poids de chacun des composants lorsqu'ils sont utilisés de façon séparée ou par rapport au poids total de la composition contenant les composants (A) et (B).

Les compositions constituées, soit par des composants (A) et (B), ou par la composition contenant les deux composants (A) et (B), peuvent également contenir tous autres adjuvants habituellement utilisés dans les compositions destinées à une application topique à utilisation cosmétique ou pharmaceutique et plus particulièrement des agents conservateurs, des agents complexants, des colorants, des agents alcalinisants ou acidifiants, des agents tensio-actifs, anioniques, cationiques, non ioniques, amphotères ou leurs mélanges, des polymères anioniques, cationiques, non ioniques ou amphotères, ainsi que leurs mélanges.

Le pH de ces compositions peut varier entre 4 et 9.

Ces compositions peuvent également être pressurisées dans des dispositifs aérosols en présence d'un agent propulseur.

Dans le composant (B), les dérivés de pyrimidine de formule (I) peuvent être présents, soit sous forme dissoute dans les milieux physiologiquement acceptables, ou alors en totalité ou partiellement en suspension dans ce milieu, et plus particulièrement sous forme de particules ayant une granulométrie inférieure à 80 $\mu$m, de préférence inférieure à 20 $\mu$m, et en particulier inférieure à 5 $\mu$m.

Une forme de réalisation de l'invention consiste à utiliser l'association conforme à l'invention sous forme d'une composition unique contenant les composants (A) et (B).

Une forme particulièrement préférée de la réalisation de l'invention consiste à conserver, dans des dispositifs séparés, les composants (A) et (B), et à préparer la composition contenant ces deux composants tout juste avant application.

Une autre forme de réalisation consiste enfin à appliquer les composants (A) et (B) de façon séparée, soit simultanément, soit de façon successive ou décalée dans le temps. Dans cette forme de réalisation, la composition (A) étant appliquée de préférence avant la composition (B).

L'association conforme à l'invention peut être conditionnée, dans ce cas, en particulier, dans un dispositif à plusieurs compartiments appelés "kit" ou nécessaire, dont un premier compartiment contient le composant (A) renfermant l'agent anti-inflammatoire non stéroïdien défini ci-dessus et le second compartiment contient le composant (B) à base du dérivé de pyrimidine de formule (I).

Ces compositions sont appliquées de préférence sur les cheveux ou le cuir chevelu. Elles peuvent s'appliquer, par exemple, après lavage du cuir chevelu et des cheveux à l'aide d'un shampooing.

Une forme de réalisation préférée consiste à appliquer 1 à 2 grammes de la composition selon l'invention sur la zone alopécique à une fréquence de une à deux applications par jour pendant 1 à 7 jours par semaine et ceci pendant une durée de 1 à 6 mois.

Conformément à l'invention, on peut également appliquer la composition contenant l'agent anti-inflammatoire non stéroïdien le soir et le composant (B) le matin.

La forme de réalisation préférée consiste à appliquer successivement ou même simultanément les composants (A) et (B) en procédant à un mélange au moment de l'application.

Les dispositifs à plusieurs compartiments peuvent être équipés dans ce but d'un dispositif de mélange bien connu dans l'état de la technique.

Le procédé conforme à l'invention vise le traitement thérapeutique de la chute des cheveux dans la mesure où il a une action sur le dysfonctionnement des mécanismes biologiques à l'origine de la pousse des cheveux.

L'invention a donc également pour objet un procédé de préparation d'un médicament éventuellement à deux composants destiné à traiter la chute des cheveux et en particulier l'alopécie.

Ce procédé peut également être considéré comme un procédé de traitement cosmétique des cheveux dans la mesure où il améliore leur aspect.

Les exemples suivants sont destinés à illustrer l'invention sans pour autant présenter un caractère limitatif.

EXEMPLE 1

On conditionne en kit deux compositions (A) et (B) renfermant respectivement :

| Composition (A) : | |
|---|---|
| - Piroxicam<br>- Propylèneglycol<br>- Alcool éthylique<br>- Eau | 0,02 g<br>20,00 g<br>50,00 g<br>qsp 100,00 g |
| Composition (B) : | |
| - Minoxidil<br>- Propylèneglycol<br>- Alcool éthylique<br>- Eau | 1,50 g<br>20,00 g<br>50,00 g<br>qsp 100,00 g |

On applique sur les parties alopéciques du cuir chevelu le mélange extemporané des deux compositions (A) et (B).

EXEMPLE 2

On conditionne en kit deux compositions (A) et (B) renfermant respectivement :

| Composition (A) : | |
|---|---|
| - Diflunisal<br>- Propylèneglycol<br>- Alcool éthylique<br>- Eau | 0,50 g<br>20,00 g<br>50,00 g<br>qsp 100,00 g |
| Composition (B) : | |
| - Minoxidil<br>- Alcool éthylique<br>- Propylèneglycol | 1,80 g<br>95,00 g<br>qsp 100,00 g |

On applique sur les parties alopéciques du cuir chevelu les compositions (A) et (B) en succession décalée dans le temps. On applique la composition (A) le matin et la composition (B) le soir.

EXEMPLE 3

On prépare la composition suivante :

| | |
|---|---|
| - Diclofénac | 0,08 g |
| - Minoxidil | 1,40 g |
| - Alcool éthylique | 50,00 g |
| - Propylèneglycol | 20,00 g |
| - Gomme de xanthane vendue par la Société KELCO sous la dénomination "KELTROL T" | 0,80 g |
| - Eau | qsp 100,00 g |

On applique cette composition comme indiqué dans l'exemple 2.

EXEMPLE 4

On conditionne en kit deux compositions (A) et (B) renfermant respectivement :

| Composition (A) : | |
|---|---|
| - Acide flufénamique | 0,15 g |
| - Propylèneglycol | 20,00 g |
| - Alcool éthylique | 50,00 g |
| - Eau | qsp 100,00 g |
| Composition (B) : | |
| - Minoxidil | 2,50 g |
| - Propylèneglycol | 20,00 g |
| - Alcool éthylique | 50,00 g |
| - Hydroxypropyl cellulose vendue par la Société HERCULES sous la dénomination "KLUCEL G" | 2,00 g |
| - Eau | qsp 100,00 g |

On applique sur les parties alopéciques du cuir chevelu les deux compositions (A) et (B) en succession décalée dans le temps : composition (A) le matin, composition (B) le soir.

EXEMPLE 5

On conditionne en kit deux compositions (A) et (B) renfermant respectivement :

| Composition (A) : | |
|---|---|
| - Acide niflumique | 0,30 g |
| - Propylèneglycol | 20,00 g |
| - Alcool éthylique | 50,00 g |
| - Gomme de xanthane vendue par la Société KELCO sous la dénomination "KELTROL T" | 0,70 g |
| - Eau | qsp 100,00 g |
| Composition (B) : | |
| - Minoxidil | 2,00 g |
| - Propylèneglycol | 20,00 g |
| - Alcool éthylique | 50,00 g |
| - Gomme de xanthane vendue par la Société KELCO sous la dénomination "KELTROL T" | 0,70 g |
| - Eau | qsp 100,00 g |

On applique sur les parties alopéciques du cuir chevelu les compositions (A) et (B) en succession décalée dans le temps, en alternance journalière : premier jour application de la composition (A), second jour application de la composition (B).

EXEMPLE 6

On prépare la composition suivante :

| | |
|---|---|
| - Acide niflumique | 3,75 g |
| - Minoxidil | 0,625 g |
| - Propylène glycol distillé | 6,45 g |
| - Alcool éthylique absolu | qsp 100,00 g |

Cette lotion, appliquée quotidiennement sur les zones alopéciques du cuir chevelu engendre un effet positif sur la repousse des cheveux et la diminution de leur chute.

EXEMPLE 7

| | |
|---|---|
| - Buféxamac | 3,00 g |
| - Minoxidil | 1,00 g |
| - Propylène glycol distillé | 6,45 g |
| - Alcool éthylique absolu | qsp 100,00 g |

EXEMPLE 8

On conditionne en kit deux compositions (A) et (B) renfermant respectivement :

| | |
|---|---|
| Composition (A) : | |
| - Acide flufénamique | 5,00 g |
| - Propylèneglycol distillé | 6,45 g |
| - Alcool éthylique absolu | qsp 100,00 g |
| Composition (B) : | |
| - Minoxidil | 1,00 g |
| - Propylèneglycol distillé | 6,45 g |
| - Alcool éthylique absolu | qsp 100,00 g |

dont on mélange équipondéralement (50/50) lesdites compositions avant l'application.

EXEMPLE 9

On prépare la composition suivante :

| | |
|---|---|
| - Diflunisal | 4,00 g |
| - Minoxidil | 0,8 g |
| - Propylène glycol distillé | 6,45 g |
| - Alcool éthylique absolu | qsp 100,00 g |

EXEMPLE 10

On conditionne en kit deux compositions (A) et (B) renfermant respectivement :

| Composition (A) : | |
|---|---|
| - Diclofénac<br>- Propylèneglycol distillé<br>- Alcool éthylique absolu | 1,4 g<br>6,45 g<br>qsp 100,00 g |
| Composition (B) : | |
| - Minoxidil<br>- Propylèneglycol distillé<br>- Alcool éthylique absolu | 1,00 g<br>6,45 g<br>qsp 100,00 g |

On applique ces compositons de façon décalée dans le temps, l'une le matin (A), l'autre le soir (B).

EXEMPLE 11

On conditionne en kit deux compositions (A) et (B) renfermant respectivement :

| Composition (A) : | |
|---|---|
| - Fenbufène<br>- Propylèneglycol distillé<br>- Alcool éthylique absolu | 3,00 g<br>6,45 g<br>qsp 100,00 g |
| Composition (B) : | |
| - Minoxidil<br>- Propylèneglycol distillé<br>- Alcool éthylique absolu | 0,70 g<br>6,45 g<br>qsp 100,00 g |

On applique (A) puis (B) ou vice versa, en succession immédiate sur les zones alopéciques du cuir chevelu.

EXEMPLE 12

On conditionne en kit deux compositions (A) et (B) renfermant respectivement :

| Composition (A) : | |
|---|---|
| - Fénoprofène<br>- Propylèneglycol distillé<br>- Alcool éthylique absolu | 2,5 g<br>6,45 g<br>qsp 100,00 g |
| Composition (B) : | |
| - Minoxidil<br>- Propylèneglycol distillé<br>- Alcool éthylique absolu | 1,50 g<br>6,45 g<br>qsp 100,00 g |

On applique les compositons (A), et (B) en alternance journalière.

EXEMPLE 13

On conditionne en kit deux compositions (A) et (B) renfermant respectivement :

| Composition (A) : | |
|---|---|
| - Piroxicam | 0,4 g |
| - Monométhyléther de propylèneglycol | 75,0 g |
| - Alcool éthylique absolu | qsp 100,00 g |
| **Composition (B) :** | |
| - Minoxidil | 2,50 g |
| - Propylèneglycol distillé | 6,45 g |
| - Alcool éthylique absolu | qsp 100,00 g |

On applique ces compositons sur les zones alopéciques du cuir chevelu en succession décalée dans le temps l'une le matin (A), l'autre le soir (B) ou vice versa.

## Revendications

**Revendications pour les Etats contractants suivants : AT, BE, CH, DE, ES, FR, GB, IT, LI, NL, SE**

**1.** Association destinée à induire et à stimuler la croissance des cheveux et diminuer leur chute, caractérisée par le fait qu'elle comprend :

a/ un composant (A) contenant dans un milieu physiologiquement acceptable au moins un agent anti-inflammatoire non stéroïdien choisi parmi les Oxicams, l'acide niflumique, le diclofenac, le diflunisal, l'acide flufénamique, le buféxamac, le fenbufène, le fénoprofène ainsi que leurs sels et esters physiologiquement acceptables;

b/ un composant (B) contenant dans un milieu physiologiquement acceptable au moins un dérivé de pyrimidine répondant à la formule :

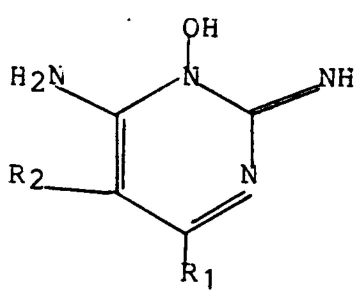

(I)

dans laquelle $R_1$ désigne un groupement

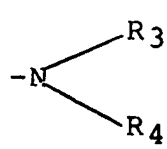

dans lequel $R_3$ et $R_4$, indépendamment l'un de l'autre, désignent hydrogène, un groupement alkyle, alcényle, alkylaryle, cycloalkyle, $R_3$ et $R_4$ peuvent également former un hétérocycle avec l'atome d'azote auquel ils sont liés, choisi parmi les groupements aziridinyle, azétidinyle, pyrrolidinyle, pipéridinyle, hexahydroazépinyle, heptaméthylèneimine, octaméthylèneimine, morpholine et alkyl-(inférieur)-4 pipérazidinyle, les groupements hétérocycliques pouvant être substitués sur es atomes de carbone par un à trois groupements alkyle inférieur, hydroxy ou alcoxy; le groupement $R_2$ est choisi parmi un atome d'hydrogène, un groupement alkyle, alcényle, alkylalcoxy, cycloalkyle, aryle, alkylaryle, arylalkyle, alkylarylalkyle, alcoxyarylalkyle ou haloarylalkyle, ainsi que les sels d'addition d'acides physiologiquement acceptables, les composants (A) et (B) faisant partie d'une même composition ou étant destinés à être utilisés séparément, soit simultanément, soit de façon successive ou décalée dans le temps sur les cheveux et le cuir chevelu.

**2.** Association selon la revendication 1, caractérisée par le fait que l'Oxicam est constitué par le

Piroxicam.

3. Association selon l'une quelconque des revendications 1 ou 2, caractérisée par le fait que le composé de formule (I) est constitué par l'amino-6 dihydro-1,2 hydroxy-1 imino-2 pipéridino-4 pyrimidine ou "Minoxidil".

4. Association selon l'une quelconque des revendications 1 à 3, caractérisée par le fait que l'agent anti-inflammatoire non stéroïdien est utilisé dans le composant (A) dans des proportions comprises entre 0,01 et 5% en poids, de préférence entre 0,05 et 3% et plus particulièrement entre 0,05 et 2% en poids; que le dérivé de pyrimidine de formule (I) est utilisé dans le composant (B) dans des proportions comprises entre 0,05 et 10% en poids, de préférence entre 0,05 et 5% en poids et en particulier entre 0,5 et 4% en poids.

5. Association selon l'une quelconque des revendications 1 à 4, caractérisée par le fait que les composants (A) et (B) sont utilisés dans des compositions uniques contenant l'agent anti-inflammatoire non stéroïdien dans des proportions comprises entre 0,01 et 3% en poids par rapport au poids total de la composition, de préférence entre 0,02 et 2% et en particulier entre 0,02 et 1%, et que le dérivé de pyrimidine de formule (I) est utilisé dans des proportions comprises entre 0,05 et 6% en poids par rapport au poids total de la composition, de préférence entre 0,1 et 5% et en particulier entre 0,5 et 3% en poids.

6. Association selon l'une quelconque des revendications 1 à 5, caractérisée par le fait que le milieu physiologiquement acceptable est constitué par l'eau, un mélange d'eau ou d'un ou plusieurs solvants organiques ou par un ou plusieurs solvants organiques pharmaceutiquement ou cosmétiquement acceptables.

7. Association selon l'une quelconque des revendications 1 à 5, caractérisée par le fait que le milieu physiologiquement acceptable est anhydre et contient des solvants organiques cosmétiquement ou pharmaceutiquement acceptables.

8. Association selon la revendication 6 ou 7, caractérisée par le fait que les solvants sont choisis parmi les alcools inférieurs en $C_1$-$C_4$, les alkylèneglycols, les alkyléthers de mono- et de dialkylèneglycol.

9. Association selon l'une quelconque des revendications 1 à 8, caractérisée par le fait que l'un au moins des milieux physiologiquement acceptables des composants (A) et (B) est épaissi au moyen d'agents épaississants et/ou gélifiants.

10. Association selon l'une quelconque des revendications 1 à 9, caractérisée par le fait que l'un au moins des composants (A) et/ou (B) contient également au moins un adjuvant cosmétiquement ou pharmaceutiquement acceptable choisi parmi des agents conservateurs, des agents complexants, des colorants, des agents alcalinisants ou acidifiants, des agents tensio-actifs, anioniques, non ioniques ou amphotères, ainsi que leurs mélanges, des polymères anioniques, cationiques, non ioniques ou amphotères ainsi que leurs mélanges.

11. Dispositif à plusieurs compartiments ou "kit", caractérisé par le fait qu'il comprend dans un premier compartiment le composant (A) contenant dans un milieu physiologiquement acceptable au moins un agent anti-inflammatoire non stéroïdien choisi parmi les Oxicams, l'acide niflumique, le diclofénac, le diflunisal, l'acide flufénamique, le buféxamac, le fenbufène, le fénoprofène, et qui est défini dans l'une quelconque des revendications 1 à 3, et dans un second compartiment le composant (B) contenant dans un milieu physiologiquement acceptable un dérivé de pyrimidine répondant à la formule (I).

12. Association selon l'une quelconque des revendications 1 à 10 pour son application comme médicament dans le traitement de la chute des cheveux.

13. Utilisation de l'association selon l'une quelconque des revendications 1 à 10 pour la préparation d'un médicament destiné à traiter la chute des cheveux.

14. Procédé de traitement cosmétique des cheveux ou du cuir chevelu, comprenant l'application de

l'association définie dans l'une quelconque des revendications 1 à 10.

**Revendications pour l'Etat contractant suivant : GR**

1. Association destinée à induire et à stimuler la croissance des cheveux et diminuer leur chute, caractérisée par le fait qu'elle comprend :
   a/ un composant (A) contenant dans un milieu physiologiquement acceptable au moins un agent anti-inflammatoire non stéroïdien choisi parmi les Oxicams, l'acide niflumique, le diclofenac, le diflunisal, l'acide flufénamique, le bufexamac, le fenbufène, le fénoprofène ainsi que leurs sels et esters physiologiquement acceptables;
   b/ un composant (B) contenant dans un milieu physiologiquement acceptable au moins un dérivé de pyrimidine répondant à la formule :

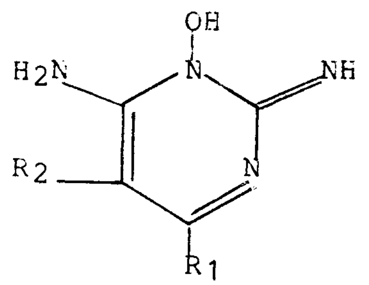

(I)

dans laquelle $R_1$ désigne un groupement

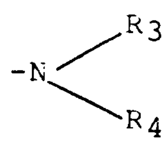

dans lequel $R_3$ et $R_4$, indépendamment l'un de l'autre, désignent hydrogène, un groupement alkyle, alcényle, alkylaryle, cycloalkyle, $R_3$ et $R_4$ peuvent également former un hétérocycle avec l'atome d'azote auquel ils sont liés, choisi parmi les groupements aziridinyle, azétidinyle, pyrrolidinyle, pipéridinyle, hexahydroazépinyle, heptaméthylèneimine, octaméthylèneimine, morpholine et alkyl-(inférieur)-4 pipérazidinyle, les groupements hétérocycliques pouvant être substitués sur es atomes de carbone par un à trois groupements alkyle inférieur, hydroxy ou alcoxy; le groupement $R_2$ est choisi parmi un atome d'hydrogène, un groupement alkyle, alcényle, alkylalcoxy, cycloalkyle, aryle, alkylaryle, arylalkyle, alkylarylalkyle, alcoxyarylalkyle ou haloarylalkyle, ainsi que les sels d'addition d'acides physiologiquement acceptables, les composants (A) et (B) faisant partie d'une même composition ou étant destinés à être utilisés séparément, soit simultanément, soit de façon successive ou décalée dans le temps sur les cheveux et le cuir chevelu.

2. Association selon la revendication 1, caractérisée par le fait que l'Oxicam est constitué par le Piroxicam.

3. Association selon l'une quelconque des revendications 1 ou 2, caractérisée par le fait que le composé de formule (I) est constitué par l'amino-6 dihydro-1,2 hydroxy-1 imino-2 pipéridino-4 pyrimidine ou "Minoxidil".

4. Association selon l'une quelconque des revendications 1 à 3, caractérisée par le fait que l'agent anti-inflammatoire non stéroïdien est utilisé dans le composant (A) dans des proportions comprises entre 0,01 et 5% en poids, de préférence entre 0,05 et 3% et plus particulièrement entre 0,05 et 2% en poids; que le dérivé de pyrimidine de formule (I) est utilisé dans le composant (B) dans des proportions comprises entre 0,05 et 10% en poids, de préférence entre 0,05 et 5% en poids et en particulier entre 0,5 et 4% en poids.

**5.** Association selon l'une quelconque des revendications 1 à 4, caractérisée par le fait que les composants (A) et (B) sont utilisés dans des compositions uniques contenant l'agent anti-inflammatoire non stéroïdien dans des proportions comprises entre 0,01 et 3% en poids par rapport au poids total de la composition, de préférence entre 0,02 et 2% et en particulier entre 0,02 et 1%, et que le dérivé de pyrimidine de formule (I) est utilisé dans des proportions comprises entre 0,05 et 6% en poids par rapport au poids total de la composition, de préférence entre 0,1 et 5% et en particulier entre 0,5 et 3% en poids.

**6.** Association selon l'une quelconque des revendications 1 à 5, caractérisée par le fait que le milieu physiologiquement acceptable est constitué par l'eau, un mélange d'eau ou d'un ou plusieurs solvants organiques ou par un ou plusieurs solvants organiques pharmaceutiquement ou cosmétiquement acceptables.

**7.** Association selon l'une quelconque des revendications 1 à 5, caractérisée par le fait que le milieu physiologiquement acceptable est anhydre et contient des solvants organiques cosmétiquement ou pharmaceutiquement acceptables.

**8.** Association selon la revendication 6 ou 7, caractérisée par le fait que les solvants sont choisis parmi les alcools inférieurs en $C_1$-$C_4$, les alkylèneglycols, les alkyléthers de mono- et de dialkylèneglycol.

**9.** Association selon l'une quelconque des revendications 1 à 8, caractérisée par le fait que l'un au moins des milieux physiologiquement acceptables des composants (A) et (B) est épaissi au moyen d'agents épaississants et/ou gélifiants.

**10.** Association selon l'une quelconque des revendications 1 à 9, caractérisée par le fait que l'un au moins des composants (A) et/ou (B) contient également au moins un adjuvant cosmétiquement ou pharmaceutiquement acceptable choisi parmi des agents conservateurs, des agents complexants, des colorants, des agents alcalinisants ou acidifiants, des agents tensio-actifs, anioniques, non ioniques ou amphotères, ainsi que leurs mélanges, des polymères anioniques, cationiques, non ioniques ou amphotères ainsi que leurs mélanges.

**11.** Dispositif à plusieurs compartiments ou "kit", caractérisé par le fait qu'il comprend dans un premier compartiment le composant (A) contenant dans un milieu physiologiquement acceptable au moins un agent anti-inflammatoire non stéroïdien choisi parmi les Oxicams, l'acide niflumique, le diclofénac, le diflunisal, l'acide flufénamique, le buféxamac, le fenbufène, le fénoprofène, et qui est défini dans l'une quelconque des revendications 1 à 3, et dans un second compartiment le composant (B) contenant dans un milieu physiologiquement acceptable un dérivé de pyrimidine répondant à la formule (I).

## Claims

### Claims for the following Contracting States : AT, BE, CH, DE, FR, GB, IT, LI, NL, SE

**1.** Combination for inducing and stimulating hair growth and for reducing its loss, characterised in that it comprises:

a/ a component (A) containing in a physiologically acceptable medium at least one nonsteroidal antiinflammatory agent chosen from Oxicams, niflumic acid, diclofenac, diflunisal, flufenamic acid, bufexamac, fenbufen and fenoprofen as well as their physiologically acceptable salts and esters;

b/ a component (B) containing in a physiologically acceptable medium at least one pyrimidine derivative of the formula:

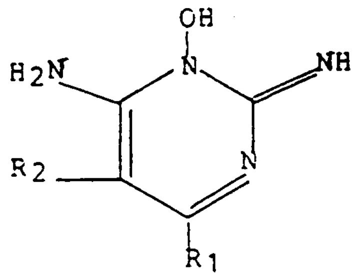

(I)

in which $R_1$ denotes a group

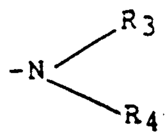

in which $R_3$ and $R_4$, independently of each other, denote hydrogen or an alkyl, alkenyl, alkylaryl or cycloalkyl group, $R_3$ and $R_4$ may also form a heterocycle with the nitrogen atom to which they are attached, chosen from the following groups: aziridinyl, azetidinyl, pyrrolidinyl, piperidinyl, hexahydroazepinyl, heptamethyleneimine, octamethyleneimine, morpholine and 4-(lower alkyl)-piperazidinyl, it being possible for the heterocyclic groups to be substituted on the carbon atoms by one to three lower alkyl or hydroxy or alkoxy groups; the $R_2$ group is chosen from a hydrogen atom, an alkyl, alkenyl, alkylalkoxy, cycloalkyl, aryl, alkylaryl, arylalkyl, alkylarylalkyl, alkoxyarylalkyl or haloarylalkl group, as well as the addition salts of physiologically acceptable acids, the components (A) and (B) being part of the same composition or being intended to be used separately on the hair and the scalp either simultaneously or successively or spaced out over time.

**2.** Combination according to Claim 1, characterised in that the Oxicam consists of Piroxicam.

**3.** Combination according to either of Claims 1 and 2, characterised in that the compound of formula (I) consists of 6-amino-1,2-dihydro-1-hydroxy-2-imino-4-piperidinopyrimidine or "Minoxidil".

**4.** Combination according to any one of Claims 1 to 3, characterised in that the nonsteroidal antiinflammatory agent is used in the component (A) in proportions of between 0.01 and 5% by weight, preferably between 0.05 and 3% and more particularly between 0.05 and 2% by weight; in that the pyrimidine derivative of formula (I) is used in the component (B) in proportions of between 0.05 and 10% by weight, preferably between 0.05 and 5% by weight and in particular between 0.5 and 4% by weight.

**5.** Combination according to any one of Claims 1 to 4, characterised in that the components (A) and (B) are used in single compositions containing the nonsteroidal antiinflammatory agent in proportions of between 0.01 and 3% by weight relative to the total weight of the composition, preferably between 0.02 and 2% and in particular between 0.02 and 1%, and in that the pyrimidine derivative of formula (I) is used in proportions of between 0.05 and 6% by weight relative to the total weight of the composition, preferably between 0.1 and 5% and in particular between 0.5 and 3% by weight.

**6.** Combination according to any one of Claims 1 to 5, characterised in that the physiologically acceptable medium consists of water, a mixture of water or of one or more organic solvents or of one or more pharmaceutically or cosmetically acceptable organic solvents.

**7.** Combination according to any one of Claims 1 to 5, characterised in that the physiologically acceptable medium is anhydrous and contains cosmetically or pharmaceutically acceptable organic solvents.

**8.** Combination according to Claim 6 or 7, characterised in that the solvents are chosen from $C_1$-$C_4$ lower alcohols, alkylene glycols and mono- and dialkylene glycol alkyl ethers.

**9.** Combination according to any one of Claims 1 to 8, characterised in that at least one of the pharmaceutically acceptable media of the components (A) and (B) is thickened by means of thickening and/or gelling agents.

**10.** Combination according to any one of Claims 1 to 9, characterised in that at least one of the components (A) and/or (B) also contains at least one cosmetically or pharmaceutically acceptable adjuvant chosen from preserving agents, complexing agents, dyes, alkalinising or acidifying agents, anionic, nonionic or amphoteric surface-active agents as well as their mixtures, anionic, cationic, nonionic or amphoteric polymers as well as their mixtures.

**11.** Multicompartment device or "kit", characterised in that it comprises in a first compartment the component (A) containing in a physiologically acceptable medium at least one nonsteroidal antiinflammatory agent chosen from Oxicams, niflumic acid, diclofenac, diflunisal, flufenamic acid, bufexamac, fenbufen and fenoprofen, and which is defined in any one of Claims 1 to 3, and, in a second compartment, the component (B) containing in a physiologically acceptable medium a pyrimidine derivative of the formula (I).

**12.** Combination according to any one of Claims 1 to 10 to be applied as medicinal product in the treatment of hair loss.

**13.** Use of the combination according to any one of Claims 1 to 10 for the preparation of a medicinal product for treating hair loss.

**14.** Process of cosmetic treatment of hair or the scalp, comprising the application of the combination defined in any one of Claims 1 to 10.

**Claims for the following Contracting State : GR**

**1.** Combination for inducing and stimulating hair growth and for reducing its loss, characterised in that it comprises:

a/ a component (A) containing in a physiologically acceptable medium at least one nonsteroidal antiinflammatory agent chosen from Oxicams, niflumic acid, diclofenac, diflunisal, flufenamic acid, bufexamac, fenbufen and fenoprofen as well as their physiologically acceptable salts and esters;

b/ a component (B) containing in a physiologically acceptable medium at least one pyrimidine derivative of the formula:

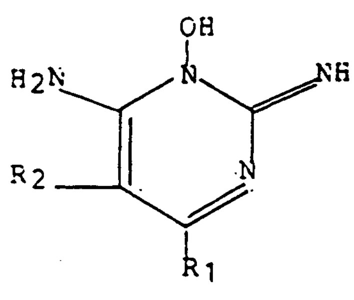

(I)

in which $R_1$ denotes a group

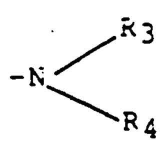

in which $R_3$ and $R_4$, independently of each other, denote hydrogen or an alkyl, alkenyl, alkylaryl or cycloalkyl group, $R_3$ and $R_4$ may also form a heterocycle with the nitrogen atom to which they are attached, chosen from the following groups: aziridinyl, azetidinyl, pyrrolidinyl, piperidinyl, hexahydroazepinyl, heptamethyleneimine, octamethyleneimine, morpholine and 4-(lower alkyl)-piperazidinyl, it being possible for the heterocyclic groups to be substituted on the carbon atoms by one to three lower alkyl or hydroxy or alkoxy groups; the $R_2$ group is chosen from a hydrogen atom, an alkyl, alkenyl, alkylalkoxy, cycloalkyl, aryl, alkylaryl, arylalkyl, alkylarylalkyl, alkoxyarylalkyl or haloarylalkyl group, as well as the addition salts of physiologically acceptable acids, the components (A) and (B) being part of the same composition or being intended to be used separately on the hair and the scalp either simultaneously or successively or spaced out over time.

**2.** Combination according to Claim 1, characterised in that the Oxicam consists of Piroxicam.

**3.** Combination according to either of Claims 1 and 2, characterised in that the compound of formula (I) consists of 6-amino-1,2-dihydro-1-hydroxy-2-imino-4-piperidinopyrimidine or "Minoxidil".

**4.** Combination according to any one of Claims 1 to 3, characterised in that the nonsteroidal antiinflammatory agent is used in the component (A) in proportions of between 0.01 and 5% by weight, preferably between 0.05 and 3% and more particularly between 0.05 and 2% by weight; in that the pyrimidine derivative of formula (I) is used in the component (B) in proportions of between 0.05 and 10% by weight, preferably between 0.05 and 5% by weight and in particular between 0.5 and 4% by weight.

**5.** Combination according to any one of Claims 1 to 4, characterised in that the components (A) and (B) are used in single compositions containing the nonsteroidal antiinflammatory agent in proportions of between 0.01 and 3% by weight relative to the total weight of the composition, preferably between 0.02 and 2% and in particular between 0.02 and 1%, and in that the pyrimidine derivative of formula (I) is used in proportions of between 0.05 and 6% by weight relative to the total weight of the composition, preferably between 0.1 and 5% and in particular between 0.5 and 3% by weight.

**6.** Combination according to any one of Claims 1 to 5, characterised in that the physiologically acceptable medium consists of water, a mixture of water or of one or more organic solvents or of one or more pharmaceutically or cosmetically acceptable organic solvents.

**7.** Combination according to any one of Claims 1 to 5, characterised in that the physiologically acceptable medium is anhydrous and contains cosmetically or pharmaceutically acceptable organic solvents.

**8.** Combination according to Claim 6 or 7, characterised in that the solvents are chosen from $C_1$-$C_4$ lower alcohols, alkylene glycols and mono- and dialkylene glycol alkyl ethers.

**9.** Combination according to any one of Claims 1 to 8, characterised in that at least one of the pharmaceutically acceptable media of the components (A) and (B) is thickened by means of thickening and/or gelling agents.

**10.** Combination according to any one of Claims 1 to 9, characterised in that at least one of the components (A) and/or (B) also contains at least one cosmetically or pharmaceutically acceptable adjuvant chosen from preserving agents, complexing agents, dyes, alkalinising or acidifying agents, anionic, nonionic or amphoteric surface-active agents as well as their mixtures, anionic, cationic, nonionic or amphoteric polymers as well as their mixtures.

**11.** Multicompartment device or "kit", characterised in that it comprises in a first compartment the component (A) containing in a physiologically acceptable medium at least one nonsteroidal antiinflammatory agent chosen from Oxicams, niflumic acid, diclofenac, diflunisal, flufenamic acid, bufexamac, fenbufene and fenoprofene, and which is defined in any one of Claims 1 to 3, and, in a second compartment, the component (B) containing in a physiologically acceptable medium a pyrimidine derivative of the formula (I).

**Patentansprüche**

**Patentansprüche für folgende Vertragsstaaten : AT, BE, CH, DE, FR, GB, IT, LI, NL, SE**

**1.** Zusammensetzung zum Induzieren und Stimulieren des Haarwachstums und zur Verminderung des Haarausfalls, dadurch gekennzeichnet, daß sie umfaßt:

a/ ein Mittel (A), das in einem physiologisch akzeptablen Milieu wenigstens ein nicht-steroides entzündungshemmendes Mittel, ausgewählt unter den Oxicamen, der Nifluminsäure, dem Diclofenac, dem Diflunisal, der Flufenaminsäure, dem Bufexamac, dem Fenbufen, dem Fenoprofen, wie auch deren physiologisch akzeptablen Salzen und Estern enthält;

b/ ein Mittel (B), das in einem physiologisch akzeptablen Milieu wenigstens ein Pyrimidinderivat gemäß der Formel:

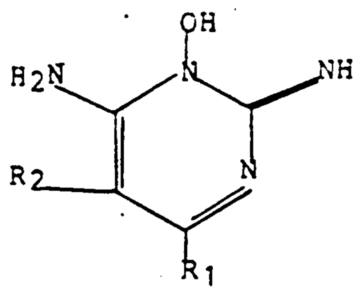

(I)

enthält, in der $R_1$ eine Gruppe

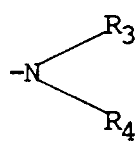

ist,

wobei $R_3$ und $R_4$ unabhängig voneinander Wasserstoff, eine Alkyl-, Alkenyl-, Alkylaryl- und Cycloalkylgruppe sein können und wobei $R_3$ und $R_4$ gleicherweise einen Heterozyklus mit dem Stickstoffatom bilden können, an welches sie gebunden sind, ausgewählt unter den Gruppen Aziridinyl, Azetidinyl, Pyrrolidinyl, Piperidinyl, Hexahydroazipenyl, Heptamethylenimin, Octamethylenimin, Morpholin und 4-Alkyl-(niedere)-piperazidinyl, wobei die heterozyklischen Gruppen an den Kohlenstoffatomen durch ein bis drei niedere Akyl-, Hydroxy- oder Alkoxygruppen substituiert sein können; wobei die Gruppe $R_2$ ausgewähltist unter einem Wasserstoffatom, einer Alkyl-, Alkenyl-, Alkylalkoxy-, Cycloalkyl-, Aryl-, Alkylaryl-, Arylalkyl-, Alkylarylalkyl-, Alcoxyarylalkyl- oder Haloarylalkylgruppe, wie auch die physiologisch akzeptablen Säureadditionssalze, wobei die Mittel (A) und (B) Bestandteil des gleichen Mittels sind oder für eine getrennte Verwendung bestimmt sind, die gleichzeitig oder aufeinanderfolgend oder zeitlich verschoben auf die Haare und auf die Kopfhaut aufgetragen werden.

**2.** Zusammensetzung nach Anspruch 1, dadurch gekennzeichnet, daß das Oxicam durch das Piroxicam gebildet ist.

**3.** Zusammensetzung nach einem der Ansprüche 1 oder 2, dadurch gekennzeichnet, daß die Verbindung der Formel (I) durch ein 6-Amino-1,2-dihydro-1-hydroxy-2-imino-4-piperidino-pyrimidin oder "Minoxidil" gebildet ist.

**4.** Zusammensetzung nach einem der Ansprüche 1 bis 3, dadurch gekennzeichnet, daß das nicht-steroide entzündungshemmende Mittel in dem Mittel (A) in Mengen verwendet wird, die zwischen 0,01 und 5 Gew.-% liegen, bevorzugt zwischen 0,05 und 3 Gew.-% und besonders bevorzugt zwischen 0,05 und 2 Gew.-%; daß das Pyrimidinderivat der Formel (I) in dem Mittel (B) in Mengen verwendet wird, die zwischen 0,05 und 10 Gew.-%, bevorzugt zwischen 0,05 und 5 Gew.-% besonders bevorzugt zwischen 0,5 und 4 Gew.-% liegen.

**5.** Zusammensetzung nach einem der Ansprüche 1 bis 4, dadurch gekennzeichnet, daß die Mittel (A) und (B) in vereinigten Mitteln verwendet werden, welche das nicht-steroide entzündungshemmende Mittel in Mengen enthalten, die zwischen 0,01 und 3 Gew.-% bezogen auf das Gesamtgewicht des Mittels, bevorzugt zwischen 0,02 und 2 Gew.-% und insbesondere zwischen 0,02 und 1 Gew.-% liegen, und daß das Pyrimidinderivat der Formel (I) in Mengen verwendet wird, die zwischen 0,05 und 6 Gew.-%, bezogen auf das Gesamtgewicht des Mittels, bevorzugt zwischen 0,1 und 5 Gew.-% und insbesondere

zwischen 0,5 und 3 Gew.-% liegen.

**6.** Zusammensetzung nach einem der Ansprüche 1 bis 5, dadurch gekennzeichnet, daß das physiologisch akzeptable Milieu durch Wasser, eine Mischung von Wasser oder eines oder mehrerer organischer Lösungsmittel oder durch oder mehrere pharmazeutisch oder kosmetisch akzeptable organische Lösungsmittel gebildet ist.

**7.** Zusammensetzung nach einem der Ansprüche 1 bis 5, dadurch gekennzeichnet, daß das physiologisch akzeptable Milieu wasserfrei ist und kosmetisch oder pharmazeutisch akzeptable organische Lösungsmittel enthält.

**8.** Zusammensetzung nach Anspruch 6 oder 7, dadurch gekennzeichnet, daß die Lösungsmittel unter niedrigen $C_1$-$C_4$-Alkoholen, den Alkylenglykolen und den Alkylethern des Mono- und des Dialkylenglykols gewählt sind.

**9.** Zusammensetzung nach einem Ansprüche 1 bis 8, dadurch gekennzeichnet, daß wenigstens eines der physiologisch akzeptablen Milieus der Mittel (A) und (B) mittels eines Verdickungsmittels oder eines Gelierungsmittels verdickt ist.

**10.** Zusammensetzung nach einem Ansprüche 1 bis 9, dadurch gekennzeichnet, daß wenigstens eines der Mittel (A) und/oder (B) weiters wenigstens ein kosmetisch oder pharmazeutisch akzeptables Adjuvans enthält, ausgewählte unter den Konservierungsmitteln, Komplexierungsmitteln, Farbstoffen, Alkalisierungs- oder Säuerungsmitteln, anionischen, nichtionischen oder amphotären oberflächenaktiven Mitteln, wie auf deren Mischungen, anionischen, kationischen, nichtionischen oder amphoteren Polymeren und deren Mischungen.

**11.** Vorrichtung mit mehreren Kompartimenten oder "Kit", dadurch gekennzeichnet, daß sie in einem ersten Kompartiment das Mittel (A) aufweist, das in einem physiologisch akzeptablen Milieu wenigstens ein nicht-steroides entzündungshemmendes Mittel enthält, ausgewählt unten den Oxicamen, der Nifluminsäure, dem Diclofenac, dem Diflunisal, der Flufenaminsäure, dem Bufexamac, dem Fenbufen, dem Fenoprofen, wie in einem der Ansprüche 1 bis 3 definiert, und daß in einem zweiten Kompartiment das Mittel (B) enthalten ist, das in einem physiologisch akzeptablen Milieu ein Pyrimidinderivat entsprechend der Formel (I) enthält.

**12.** Zusammensetzung nach einem Ansprüche 1 bis 10 zur Anwendung als Medikament für die Behandlung von Haarausfall.

**13.** Verwendung der Zusammensetzung nach einem der Ansprüche 1 bis 10 für die Herstellung eines Medikamentes zur Behandlung von Haarausfall.

**14.** Verfahren zur kosmetischen Behandlung von Haaren und Kopfhaut, welches das Auftragen der Zusammensetzung, wie in einem der Ansprüche 1 bis 10 definiert, umfaßt.

**Patentansprüche für folgenden Vertragsstaat : GR**

**1.** Zusammensetzung zum Induzieren und Stimulieren des Haarwachstums und zur Verminderung des Haarausfalls, dadurch gekennzeichnet, daß sie umfaßt:

a/ ein Mittel (A), das in einem physiologisch akzeptablen Milieu wenigstens ein nicht-steroides entzündungshemmendes Mittel, ausgewählt unten den Oxicamen, der Nifluminsäure, dem Diclofenac, dem Diflunisal, der Flufenaminsäure, dem Bufexamac, dem Fenbufen, dem Fenoprofen, wie auch deren physiologisch akzeptablen Salzen und Estern enthält;

b/ ein Mittel (B), das in einem physiologisch akzeptablen Milieu wenigstens ein Pyrimidinderivat gemäß der Formel:

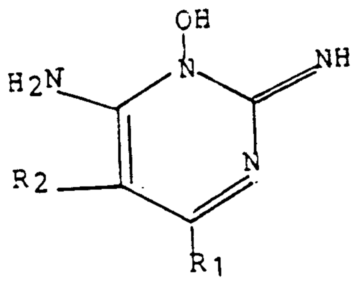

(I)

enthält, in der $R_1$ eine Gruppe

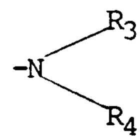 ist,

wobei $R_3$ und $R_4$ unabhängig voneinander Wasserstoff, eine Alkyl-, Alkenyl-, Alkylaryl- und Cycloalkylgruppe sein können und wobei $R_3$ und $R_4$ gleicherweise einen Heterozyklus mit dem Stickstoffatom bilden können, an welches sie gebunden sind, ausgewählt unter den Gruppen Aziridinyl, Azetidinyl, Pyrrolidinyl, Piperidinyl, Hexahydroazipenyl, Heptamethylenimin, Octamethylenimin, Morpholin und 4-Alkyl-(niedere)-piperazidinyl, wobei die heterozyklischen Gruppen an den Kohlenstoffatomen durch ein bis drei niedere Akyl-, Hydroxy- oder Alkoxygruppen substituiert sein können; wobei die Gruppe $R_2$ ausgewähltist unter einem Wasserstoffatom, einer Alkyl-, Alkenyl-, Alkylalkoxy-, Cycloalkyl-, Aryl-, Alkylaryl-, Arylalkyl-, Alkylarylalkyl-, Alcoxyarylalkyl- oder Haloarylalkylgruppe, wie auch die physiologisch akzeptablen Säureadditionssalze, wobei die Mittel (A) und (B) Bestandteil des gleichen Mittels sind oder für eine getrennte Verwendung bestimmt sind, die gleichzeitig oder aufeinanderfolgend oder zeitlich verschoben auf die Haare und auf die Kopfhaut aufgetragen werden.

**2.** Zusammensetzung nach Anspruch 1, dadurch gekennzeichnet, daß das Oxicam durch das Piroxicam gebildet ist.

**3.** Zusammensetzung nach einem der Ansprüche 1 oder 2, dadurch gekennzeichnet, daß die Verbindung der Formel (I) durch ein 6-Amino-1,2-dihydro-1-hydroxy-2-imino-4-piperidino-pyrimidin oder "Minoxidil" gebildet ist.

**4.** Zusammensetzung nach einem der Ansprüche 1 bis 3, dadurch gekennzeichnet, daß das nicht-steroide entzündungshemmende Mittel in dem Mittel (A) in Mengen verwendet wird, die zwischen 0,01 und 5 Gew.-% liegen, bevorzugt zwischen 0,05 und 3 Gew.-% und besonders bevorzugt zwischen 0,05 und 2 Gew.%; daß das Pyrimidinderivat der Formel (I) in dem Mittel (B) in Mengen verwendet wird, die zwischen 0,05 und 10 Gew-%, bevorzugt zwischen 0,05 und 5 Gew.-% und besonders bevorzugt zwischen 0,5 und 4 Gew.-% liegen.

**5.** Zusammensetzung nach einem der Ansprüche 1 bis 4, dadurch gekennzeichnet, daß die Mittel (A) und (B) in vereinigten Mitteln verwendet werden, welche das nicht-steroide entzündungshemmende Mittel in Mengen enthalten, die zwischen 0,01 und 3 Gew.-%, bezogen auf das Gesamtgewicht des Mittels, bevorzugt zwischen 0,02 und 2 Gew.-% und insbesondere zwischen 0,02 und 1 Gew.-% liegen, und daß das Pyrimidinderivat der Formel (I) in Mengen verwendet wird, die zwischen 0,05 und 6 Gew.-%, bezogen auf das Gesamtgewicht des Mittels, bevorzugt zwischen 0,1 und 5 Gew.-% und insbesondere zwischen 0,5 und 3 Gew.-% liegen.

**6.** Zusammensetzung nach einem der Ansprüche 1 bis 5, dadurch gekennzeichnet, daß das physiologisch akzeptable Milieu durch Wasser, eine Mischung von Wasser oder eines oder mehrerer organischer Lösungsmittel oder durch ein oder mehere pharmazeutisch oder kosmetisch akzeptable organische Lösungsmittel gebildet ist.

**7.** Zusammensetzung nach einem der Ansprüche 1 bis 5, dadurch gekennzeichnet, daß das physiologisch akzeptable Milieu wasserfrei ist und kosmetisch oder pharmazeutisch akzeptable organische Lösungsmittel enthält.

**8.** Zusammensetzung nach Anspruch 6 oder 7, dadurch gekennzeichnet, daß die Lösungsmittel unter niedrigen $C_1$-$C_4$-Alkoholen, den Alkylenglykolen und den Alkylethern des Mono- und des Dialkylenglykols gewählt sind.

**9.** Zusammensetzung nach einem der Ansprüche 1 bis 8, dadurch gekennzeichnet, daß wenigstens eines der physiologisch akzeptablen Milieus der Mittel (A) und (B) mittels eines Verdickungsmittels oder eines Gelierungsmittels verdickt ist.

**10.** Zusammensetzung nach einem der Ansprüche 1 bis 9, dadurch gekennzeichnet, daß wenigstens eines der Mittel (A) und/oder (B) weiters wenigstens ein kosmetisch oder pharmazeutisch akzeptables Adjuvans enthält, ausgewählt unter den Konservierungsmitteln, Komplexierungsmitteln, Farbstoffen, Alkalisierungs- oder Säuerungsmitteln, anionischen, nichtionischen oder amphotären oberflächenaktiven Mitteln, wie auch den Mischungen, anionischen, kationischen, nichtionischen oder amphoteren Polymeren und deren Mischungen.

**11.** Vorrichtung mit mehreren Kompartimenten oder "Kit", dadurch gekennzeichnet, daß sie in einem ersten Kompartiment das Mittel (A) aufweist, das in einem physiologisch akzeptablen Milieu wenigstens ein nicht-steroides entzündungshemmendes Mittel enthält, ausgewählt unter den Oxicamen, der Nifluminsäure, dem Diclofenac, dem Diflunisal, der Fluenaminsäure, dem Bufexamac, dem Fenbufen, dem Fenoprofen, wie in einem der Ansprüche 1 bis 3 definiert, und daß in einem zweiten Kompartiment das Mittel (B) enthalten ist, das in einem physiologisch akzeptablen Milieu ein Pyrimidinderivat entsprechend der Formel (I) enthält.